# EUROPÄISCHE PATENTANMELDUNG

(11) **EP 4 378 512 A1**
(43) Veröffentlichungstag der Anmeldung: **05.06.2024**
(21) Anmeldenummer: 23212921.3
(22) Anmeldetag: 29.11.2023
(51) Int. Cl.: A61M 25/00

(54) **KATHETER UND VERFAHREN ZUR HERSTELLUNG EINES SOLCHEN KATHETERS**

(30) Priorität: 01.12.2022 DE 102022212931
(71) Anmelder: B. Braun Melsungen AG, 34212 Melsungen (DE)
(72) Erfinder: Dubielzig, Egbert, 34560 Fritzlar (DE); Kahlen, Oliver, 34281 Gudensberg (DE); Weiß, André, 34302 Guxhagen (DE)
(74) Vertreter: Patentanwälte Ruff, Wilhelm, Beier, Dauster & Partner mbB

(57) **Zusammenfassung**

Katheter (1, 1a) mit einem Katheterschlauch (2, 2a), welcher ein Hauptlumen (5) und wenigstens ein Nebenlumen (6) aufweist, wobei das Hauptlumen und das wenigstens eine Nebenlumen jeweils zwischen einem proximalen Schlauchende (3) und einem distalen Schlauchende (4, 4a) längserstreckt sind, und wobei das wenigstens eine Nebenlumen eine seitliche Öffnung (7) aufweist, welche in axialer Richtung des Katheterschlauchs zwischen dem proximalen Schlauchende und dem distalen Schlauchende angeordnet und in radialer Richtung des Katheterschlauchs durch einen Schlauchmantel des Katheterschlauchs erstreckt ist, und mit einem Verschluss (V), der distal hinter der seitlichen Öffnung in das Nebenlumen eingebracht ist und dasselbe wenigstens teilweise verschließt.

Wobei der Verschluss aus einer in dem Nebenlumen ausgehärteten Verschlussmasse oder einem innerhalb des Nebenlumens expandierten thermoplastischen Elastomermaterial gebildet ist.

## Beschreibung

Die Erfindung betrifft einen Katheter mit einem Katheterschlauch, welcher ein Hauptlumen und wenigstens ein Nebenlumen aufweist, wobei das Hauptlumen und das wenigstens eine Nebenlumen jeweils zwischen einem proximalen Schlauchende und einem distalen Schlauchende längserstreckt sind, und wobei das wenigstens eine Nebenlumen eine seitliche Öffnung aufweist, welche in axialer Richtung des Katheterschlauchs zwischen dem proximalen Schlauchende und dem distalen Schlauchende angeordnet und in radialer Richtung des Katheterschlauchs durch einen Schlauchmantel des Katheterschlauchs erstreckt ist, und mit einem Verschluss, der distal hinter der seitlichen Öffnung in das Nebenlumen eingebracht ist und dasselbe wenigstens teilweise verschließt.

Ein derartiger Katheter ist im Bereich der Medizintechnik beispielsweise als mehrlumiger zentraler Venenkatheter allgemein bekannt. Der bekannte Katheter weist einen Katheterschlauch mit einem Hauptlumen und wenigstens einem Nebenlumen auf. Das Hauptlumen und das Nebenlumen sind jeweils zwischen einem proximalen Schlauchende und einem distalen Schlauchende des Katheterschlauchs längserstreckt. Das Nebenlumen weist eine in radialer Richtung durch einen Schlauchmantel des Katheterschlauchs erstreckte seitliche Öffnung auf. Bei dem bekannten Katheter ist in distaler Richtung hinter der seitlichen Öffnung ein sogenannter Mandrin in das Nebenlumen eingebracht. Der Mandrin kann beispielsweise als Stäbchen oder Schnur gestaltet sein und fungiert als Verschluss. Der Mandrin wird separat von dem Katheterschlauch gefertigt und in einem gesonderten Schritt bei der Herstellung des Katheters in proximaler oder in distaler Richtung in das Nebenlumen eingeschoben. Das Einschieben des Mandrins erfolgt manuell.

Aufgabe der Erfindung ist es, einen Katheter der eingangs genannten Art bereitzustellen, der einen einfachen Aufbau und gleichzeitig gegenüber dem Stand der Technik verbesserte Eigenschaften aufweist.

Diese Aufgabe wird dadurch gelöst, dass der Verschluss aus einer in viskosem Zustand in das Nebenlumen eingebrachten und innerhalb des Nebenlumens ausgehärteten Verschlussmasse gebildet ist. Durch die erfindungsgemäße Lösung kann auf einen Mandrin und auf dessen fertigungsseitiges Einschieben in das Nebenlumen verzichtet werden. Hierdurch werden der Aufbau des Katheters und dessen Herstellung maßgeblich vereinfacht. Dabei ist das Einbringen der (zunächst) viskosen Verschlussmasse in das Nebenlumen und das Aushärten der in das Nebenlumen eingebrachten Verschlussmasse zum Ausbilden des Verschlusses mit vergleichsweise einfachen Mitteln automatisierbar. Dies im Gegensatz zu dem aus dem Stand der Technik bekannten Einschieben des Mandrins. Die Verschlussmasse und/oder der aus derselben gebildete Verschluss ist in Radialrichtung spaltfrei in dem Nebenlumen, genauer: an deren Innenwandung, angeordnet. Durch das Aushärten kann die Verschlussmasse stoffschlüssig mit der Innenwandung des Nebenlumens verbunden sein. Infolge der spaltfreien Anordnung ist der Schlauchmantel im Bereich des Verschlusses unmittelbar kraftübertragend auf demselben abgestützt. Hierzu im Unterschied benötigen aus dem Stand der Technik bekannte Mandrins zwangsläufig ein gewisses radiales Spaltmaß, um ein fertigungsseitiges Einschieben in das Nebenlumen zu ermöglichen. Zusätzlich können herstellungsbedingte maßliche Toleranzen und somit ein zusätzliches Spaltmaß zwischen dem Mandrin und dem Nebenlumen, genauer: dessen Innenwandung, vorhanden sein. Das besagte radiale Spaltmaß kann zu einer nicht ausreichenden Abstützung des Schlauchmantels auf dem Mandrin führen. Unter Temperatur- und/oder Druckeinwirkung kann es hierdurch bei aus dem Stand der Technik bekannten Kathetern zu Einfallstellen im Bereich des Mandrins kommen. Solche Einfallstellen sind aus unterschiedlichen Gründen unerwünscht. Zudem kommt es bei aus dem Stand der Technik bekannten Kathetern mit Mandrins oftmals zur Ausbildung eines Restvolumens in Form eines Ringraums zwischen dem Mandrin und der Innenwandung des Nebenlumens. Ein solches Restvolumen ist ebenfalls unerwünscht. Innerhalb des Restvolumens kann es in der Anwendung des Katheters zur Ansammlung von stehender Flüssigkeit und damit einhergehend zu einer Ansammlung und/oder einem vermehrten Wachstum von gesundheitsgefährdenden Keimen kommen. Die Erfindung beseitigt diese Nachteile. Vorzugsweise ist die Verschlussmasse in distaler Richtung unmittelbar hinter der Öffnung in dem Nebenlumen angeordnet. Mit anderen Worten ausgedrückt, grenzt der Verschluss in axialer Richtung unmittelbar an die seitliche Öffnung an. Der Katheterschlauch ist zwischen seinem proximalen Schlauchende und seinem distalen Schlauchende längserstreckt. Vorzugsweise ist der Katheterschlauch biegeflexibel. Der Katheterschlauch weist vorzugsweise eine zylindrisch längserstreckte Grundform mit einem runden, insbesondere kreisrunden, oder ovalen Querschnitt auf. Das Hauptlumen weist vorzugsweise eine an dem proximalen Schlauchende angeordnete Eintrittsöffnung und eine an dem distalen Schlauchende und/oder einer Katheterspitze angeordnete Austrittsöffnung auf. Das Hauptlumen ist zwischen seiner Eintritts- und seiner Austrittsöffnung längserstreckt. Das wenigstens eine Nebenlumen weist vorzugsweise eine an dem proximalen Schlauchende angeordnete Eintrittsöffnung auf. Zudem weist das wenigstens eine Nebenlumen die radial durch den Schlauchmantel erstreckte seitliche Öffnung auf. Bei einer Ausgestaltung weist der Katheter eine an dem distalen Schlauchende angeordnete Katheterspitze auf, die beispielsweise konisch oder abgerundet sein kann. Die Katheterspitze kann als separates Bauteil gefertigt und hiernach an das distale Schlauchende, vorzugsweise stoffschlüssig, angefügt sein. Alternativ kann die Katheterspitze mittels Umformens des distalen Schlauchendes an den Katheterschlauch angeformt sein. Bei einer Ausgestaltung ist der Katheter ein mehrlumiger zentraler Venenkatheter. Die erfindungsgemäße Lösung ist jedoch nicht auf zentrale Venenkatheter beschränkt, sondern vorteilhaft auch auf sonstige mehrlumige Katheter anwendbar. Sofern der Katheter mehrere Nebenlumen aufweist, beispielsweise zwei, drei, vier, fünf oder mehr als fünf Nebenlumen, sind diese vorzugsweise jeweils mit einem aus der Verschlussmasse gebildeten Verschluss verschlossen.

In Ausgestaltung der Erfindung ist und/oder enthält die Verschlussmasse Cyanacrylat. Unter Cyanacrylat, Cyanoacrylat oder Alkylcyanacrylat versteht man polymerisierbare, bei Raumtemperatur flüssige chemische Verbindungen (Monomere). Solche Verbindungen werden oftmals als Klebstoff verwendet und sind unter den Bezeichnungen Sekundenkleber oder Superkleber (engl. superglue) bekannt. Bei dieser Ausgestaltung der Erfindung härtet die Verschlussmasse vorzugsweise unter der Einwirkung der Luftfeuchte aus. Dies ermöglicht eine besonders einfache Herstellung des Katheters. In ausgehärtetem Zustand weist das Cyanacrylat eine vergleichsweise hohe Beanspruchbarkeit auf. Dies geht mit unterschiedlichen Vorteilen einher.

In weiterer Ausgestaltung der Erfindung ist und/oder enthält die Verschlussmasse Silikon. Silikon ist - ebenso wie das zuvor beschriebene Cyanacrylat - unter bloßer Einwirkung der Luftfeuchte aushärtbar. Folglich erlaubt auch diese Ausgestaltung der Erfindung eine besonders einfache Herstellung des Katheters. Zudem ist Silikon im ausgehärteten Zustand elastisch, nachgiebig und/oder biegeflexibel. Dies gewährleistet, dass biegeflexible Eigenschaften des Katheterschlauchs, sofern vorhanden, durch den aus der Verschlussmasse gebildeten Verschluss nicht beeinträchtigt werden.

In weiterer Ausgestaltung der Erfindung ist und/oder enthält die Verschlussmasse ein vernetzbares Polymer, insbesondere ein Harz, ein Monopolymer und/oder ein Prepolymer. Bei dieser Ausgestaltung der Erfindung kann die Verschlussmasse demnach im weitesten Sinne als reaktive Substanz bezeichnet werden. Die reaktive Substanz kann beispielsweise unter der Einwirkung von Temperatur, UV-Licht oder dergleichen ausgehärtet werden.

Die Erfindung betrifft zudem ein Verfahren zur Herstellung eines eingangs genannten Katheters. Das erfindungsgemäße Verfahren umfasst die Schritte: Einbringen einer viskosen Verschlussmasse in das wenigstens eine Nebenlumen, wobei die viskose Verschlussmasse durch die seitliche Öffnung in das wenigstens eine Nebenlumen eingebracht wird; Aushärten der in das wenigstens eine Nebenlumen eingebrachten, viskosen Verschlussmasse, wobei der Verschluss durch die ausgehärtete Verschlussmasse gebildet wird. Hinsichtlich sich ergebender Vorteile sowie weiterer Merkmale und Ausgestaltungen des Verfahrens wird auf die Beschreibung des erfindungsgemäßen Katheters und dessen Ausgestaltung verwiesen. Das dort Gesagte gilt sinngemäß auch für das erfindungsgemäße Verfahren und dessen Ausgestaltung. Gleiches gilt in umgekehrter Weise. Das Einbringen der viskosen Verschlussmasse erfolgt durch die seitliche Öffnung und in distaler Richtung, so dass der Verschluss in distaler Richtung hinter der seitlichen Öffnung ausgebildet wird. Zum Einbringen kann die viskose Verschlussmasse beispielsweise in das wenigstens eine Nebenlumen injiziert, eingespritzt und/oder eingepresst werden. Das Einbringen kann manuell mittels eines hierfür geeigneten Werkzeugs, teil- oder vollautomatisiert erfolgen. Je nach Zusammensetzung der verwendeten Verschlussmasse kann das Aushärten beispielsweise unter Einwirkung der Luftfeuchte, einer Wärme- und/oder Strahlungseinwirkung, insbesondere UV-Lichteinwirkung, erfolgen.

Die eingangs genannte Aufgabe wird zudem dadurch gelöst, dass der Verschluss aus einem in das Nebenlumen eingebrachten und innerhalb des Nebenlumens expandierten thermoplastischen Elastomermaterials gebildet ist. Auch durch diese erfindungsgemäße Lösung können die mit einem Einschieben eines Mandrins in das Nebenlumen einhergehenden Nachteile überwunden werden. Nach Kenntnis der Erfinder sind expandierfähige thermoplastische Elastomere erst seit wenigen Jahren am Markt verfügbar. Im Bereich der Medizintechnik sind expandierfähige thermoplastische Elastomere bislang nicht weiter bekannt. Expandierfähige thermoplastische Elastomere sind auch unter der Abkürzung EP-TPE bekannt. Die Zusammensetzung solcher expandierfähigen thermoplastischen Elastomere weist ein unter Wärmezufuhr aktivierbares Treibmittel auf. Die Aktivierung des Treibmittels bewirkt eine Expansion des thermoplastischen Elastomermaterials. Bei der Expansion des EP-TPE kann dessen ursprüngliches (nicht expandiertes) Volumen um ein Vielfaches vergrößert werden. Zudem wird eine porige Morphologie ausgebildet. Im expandierten Zustand liegt das in das wenigstens eine Nebenlumen eingebrachte thermoplastische Elastomermaterial in radialer Richtung spaltfrei an der Innenwandung des wenigstens einen Nebenlumens an. Wie bereits erörtert, benötigen aus dem Stand der Technik bekannte Lösungen mit Mandrins üblicherweise ein gewisses radiales Spaltmaß, um ein fertigungsseitiges Einschieben in das Nebenlumen zu ermöglichen. Dieses radiale Spaltmaß verbleibt auch nach dem Herstellungsprozess. Aus dem besagten Spaltmaß resultieren die bereits erörterten Nachteile. Diese Nachteile werden durch die hier in Rede stehende erfindungsgemäße Lösung beseitigt. Zur weiteren Erläuterung der oberbegrifflichen Merkmale des Anspruchs 6 wird auf das zu Anspruch 1 bereits Gesagte ausdrücklich verwiesen. Die dortige Offenbarung gilt sinngemäß auch im Hinblick auf Anspruch 6. Sofern der Katheter mehrere Nebenlumen aufweist, beispielsweise zwei, drei, vier, fünf oder mehr als fünf Nebenlumen, sind diese vorzugsweise jeweils mit einem aus dem expandierten thermoplastischen Elastomermaterial gebildeten Verschluss verschlossen.

Die Erfindung betrifft zudem ein Verfahren zur Herstellung eines Katheters mit den Merkmalen des Anspruchs 6. Das besagte erfindungsgemäße Verfahren umfasst die Schritte: Einbringen eines expandierbaren thermoplastischen Elastomermaterials in das wenigstens eine Nebenlumen, wobei das expandierbare thermoplastische Elastomermaterial durch die seitliche Öffnung in das wenigstens eine Nebenlumen eingebracht wird; Expandieren des in das wenigstens eine Nebenlumen eingebrachten thermoplastischen Elastomermaterials, wobei der Verschluss durch das expandierte thermoplastische Elastomermaterial gebildet wird. Hinsichtlich sich ergebender Vorteile sowie weiterer Merkmale und Ausgestaltungen des Verfahrens wird auf die Beschreibung des Katheters nach Anspruch 6 verwiesen. Das dort Gesagte gilt sinngemäß auch für das in Rede stehende erfindungsgemäße Verfahren und dessen Ausgestaltungen. Gleiches gilt in umgekehrter Weise.

Das expandierbare thermoplastische Elastomermaterial wird in unterschiedlichen Ausgestaltungen des Verfahrens in unterschiedlicher Form in das wenigstens eine Lumen eingebracht. Bei einer Ausgestaltung werden Partikel des expandierbaren thermoplastischen Elastomermaterials in das wenigstens eine Nebenlumen eingebracht. Hierfür werden die Partikel durch die seitliche Öffnung in das wenigstens eine Nebenlumen eingefüllt. Bei weiteren Ausgestaltungen werden die besagten Partikel vor dem Einbringen in das wenigstens eine Nebenlumen verarbeitet. Je nach Art der Verarbeitung der Partikel können unterschiedliche Formlinge, Halbzeuge oder dergleichen zum Einbringen in das wenigstens eine Nebenlumen hergestellt werden. In einer Ausgestaltung werden die Partikel zu einer expandierfähigen Formschnur, Plombe oder dergleichen verarbeitet. In einer weiteren Ausgestaltung werden die Partikel in eine elastische Hülle gefüllt. Hieraus resultiert eine Art Kissengebilde. Die Formlinge und/oder Halbzeuge werden durch die seitliche Öffnung in das wenigstens eine Nebenlumen eingebracht. Das Einbringen der Partikel, Formlinge und/oder Halbzeuge kann manuell, beispielsweise mittels eines hierfür geeigneten händisch geführten Werkzeugs, halb- oder vollautomatisch erfolgen. Nach dem Einbringen wird das thermoplastische Elastomermaterial expandiert. Hierfür wird das in dem thermoplastischen Elastomermaterial enthaltene Treibmittel durch eine Wärme- oder im weitesten Sinne Energieeinwirkung aktiviert.

In weiterer Ausgestaltung umfasst das Verfahren den Schritt: Verarbeiten von Partikeln des expandierbaren thermoplastischen Elastomermaterials, wobei eine aus den Partikeln gepresste Formschnur oder ein Kissengebilde mit einer elastischen und mit den Partikeln befüllten Hülle gebildet wird, und wobei das expandierbare thermoplastische Elastomermaterial in Form der Formschnur oder des Kissengebildes durch die seitliche Öffnung in das wenigstens eine Nebenlumen eingebracht wird. Die Formschnur kann bereits vor dem Einbringen in das wenigstens eine Nebenlumen auf eine erforderliche oder gewünschte Länge zugeschnitten werden. Alternativ wird die Formschnur erst nach dem Einbringen auf Länge zugeschnitten. Hierfür wird ein nach dem Einbringen aus der seitlichen Öffnung überstehendes Ende der Formschnur abgetrennt. Die elastische Hülle kann in Form eines Beutels oder Netzes gestaltet sein. Bei einer Ausgestaltung sind die elastische Hülle und der Katheterschlauch aus demselben Kunststoffmaterial gefertigt. Hierdurch kann eine besonders vorteilhafte stoffschlüssige Verbindung zwischen der Hülle und der Innenwandung des wenigstens einen Nebenlumens erreicht werden. Bei einer weiteren Ausgestaltung sind die elastische Hülle und der Katheterschlauch aus unterschiedlichen Materialien gefertigt.

In weiterer Ausgestaltung der Erfindung umfasst das Expandieren: Aktivieren eines in dem expandierbaren thermoplastischen Elastomermaterial enthaltenen Treibmittels, wobei das Treibmittel mittels einer Energiezufuhr aktiviert wird. Die Energiezufuhr kann durch eine direkte Erwärmung und/oder Bestrahlung des mit dem expandierbaren thermoplastischen Elastomermaterial versehenen Katheterschlauchs erfolgen, beispielsweise über Heizelemente. Denkbar ist zudem ein Erwärmen über offener Flamme. Alternativ kann die Wärme über einen Wärmeträger, beispielsweise Wasserdampf, zugeführt werden. Alternativ kann die Wärme zum Aktivieren des Treibmittels über elektromagnetische Strahlung zugeführt werden. Laut Erfinder denkbar sind insbesondere eine Bestrahlung mit Mikrowellen, Infrarot- und/oder UV-Strahlung sowie Infrarot- und/oder UV-Laserstrahlen. Bei einer solchen Ausgestaltung kann es vorteilhaft sein, wenn der Katheterschlauch und/oder das in das wenigstens eine Nebenlumen eingebrachte thermoplastische Elastomermaterial einen Absorber zur Umwandlung der Strahlung in Wärme aufweist. Weiter denkbar ist ein Erwärmen mittels Induktion. In diesem Fall können als Absorber beispielsweise metallische Partikel in dem Katheterschlauch und/oder dem thermoplastischen Elastomermaterial eingebettet werden. Durch die Wärmezufuhr wird das thermoplastische Elastomermaterial zudem erweicht und fließfähig. Das erweichte und fließfähige thermoplastische Elastomermaterial wird durch das aktivierte Treibmittel expandiert. Hierdurch resultierende innere Kräfte bewirken eine mikroskopische Porenbildung, die sich in einer makroskopischen Deformation und/oder Expansion des thermoplastischen Elastomermaterials manifestiert. Nach dem Abkühlen und Erstarren verbleibt das thermoplastische Elastomermaterial in seinem expandierten Zustand. In expandiertem Zustand weist das thermoplastische Elastomermaterial eine poröse Struktur auf. Eine Morphologie dieser porösen Struktur (offen-, geschlossenporig, Porengröße, Expansionsgrad) ist durch unterschiedliche Parameter steuerbar. Als Parameter zu nennen sind insbesondere eine anteilige Menge des verwendeten bzw. vorhandenen Treibmittels ("Beladung"), eine Intensität und Dauer der Energiezufuhr, eine Abkühlgeschwindigkeit und/oder eine Keimbildungskinetik.

In weiterer Ausgestaltung der Erfindung ist der Katheterschlauch aus einem schmelzbaren Kunststoffmaterial gefertigt, und das wenigstens eine Nebenlumen ist im Bereich des distalen Schlauchendes mittels eines einstückig mit dem übrigen Katheterschlauch zusammenhängenden Materialpfropfens wenigstens teilweise verschlossen, wobei der Materialpfropfen durch aufgeschmolzenes und wieder erstarrtes Kunststoffmaterial des distalen Schlauchendes gebildet ist. Der Materialpfropfen bildet einen weiteren Verschluss des wenigstens einen Nebenlumens. Der Materialpfropfen wirkt - ähnlich wie der aus der viskosen Verschlussmasse oder dem expandierten thermoplastischen Elastomermaterial gebildete Verschluss - einem ungewollten Einfallen des Katheterschlauchs in radialer Richtung entgegen. Hierdurch werden weiter verbesserte Eigenschaften des Katheters erreicht. Der Materialpfropfen besteht aus Kunststoffmaterial, welches zuvor im Bereich des distalen Schlauchendes aufgeschmolzen, in das wenigstens eine Nebenlumen eingebracht und dort erstarrt wurde/ist. Dementsprechend ist der Materialpfropfen einstückig mit dem übrigen Katheterschlauch zusammenhängend und nicht etwa ein von dem Katheterschlauch separates Bauteil. Mit anderen Worten: Der Materialpfropfen ist ein integraler Bestandteil und/oder Abschnitt des Katheterschlauchs. Vorzugsweise erfolgt das Einbringen des geschmolzenen Kunststoffmaterials durch eine - zunächst an dem Katheterschlauch und/oder einem Schlauchhalbzeug - vorhandene distale Öffnung des Nebenlumens. Diese distale Öffnung wird durch das Ausbilden des Materialpfropfens verschlossen.

In weiterer Ausgestaltung der Erfindung ist eine Katheterspitze an das distale Schlauchende angefügt, wobei die Katheterspitze stoffschlüssig mit einer stirnendseitigen Fügefläche des Katheterschlauchs zusammengefügt ist, und wobei eine distale Stirnendfläche des Materialpfropfens einen Abschnitt der Fügefläche bildet. Die zur Verbindung mit der Katheterspitze zur Verfügung stehende stirnendseitige Fügefläche des Katheterschlauchs wird durch die distale Stirnendfläche des Materialpfropfens vergrößert. Hierdurch kann - im Vergleich zu einer Lösung ohne Materialpfropfen und/oder mit Mandrin - eine erhöhte Belastbarkeit der stoffschlüssigen Verbindung erreicht werden. Bei dieser Ausgestaltung der Erfindung ist die Katheterspitze als separat von dem Katheterschlauch gefertigtes Bauteil ausgebildet. Die stoffschlüssige Fügeverbindung ist vorzugsweise eine Klebe-, Schmelz- und/oder Schweißverbindung.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Darstellung einen erfindungsgemäßen Katheter mit einem Katheterschlauch,
- Fig. 2: in vergrößerter Längsschnittdarstellung ein distales Ende des Katheters nach Fig. 1, wobei ein Nebenlumen mit einem Verschluss verschlossen ist,
- Fig. 3 bis 9: schematisch stark vereinfachte Darstellungen zur Verdeutlichung einzelner Schritte unterschiedlicher Ausführungsformen erfindungsgemäßer Verfahren zur Herstellung des Katheters nach den Fig. 1 und 2,
- Fig. 10: in vergrößerter Längsschnittdarstellung ein distales Ende einer Ausführungsform eines erfindungsgemäßen Katheters, wobei das Nebenlumen distal mittels eines Materialpfropfens verschlossen ist, und
- Fig. 11 bis 14: weitere schematische Darstellungen zur Verdeutlichung einzelner Verfahrensschritte zur Herstellung des Katheters nach Fig. 10, wobei die Verfahrensschritte mit den anhand der Fig. 3 bis 9 verdeutlichten Verfahrensschritten kombinierbar sind.

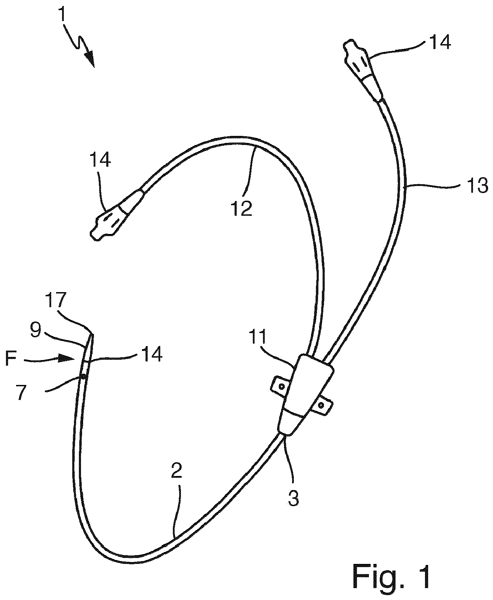

Ein Katheter 1 nach Fig. 1 ist zur Verwendung bei einer Infusionstherapie vorgesehen. Der Katheter 1 ist vorliegend ein zweilumiger zentraler Venenkatheter. Zeichnerisch nicht näher dargestellte Ausführungsformen sind auf drei-, vier-, fünf- oder mehrlumige Katheter gerichtet. Dabei ist die erfindungsgemäße Lösung nicht auf zentrale Venenkatheter beschränkt, sondern auch auf weitere medizinische Katheter mit einem mehrlumigen Katheterschlauch anwendbar.

Der Katheter 1 weist einen Katheterschlauch 2 auf.

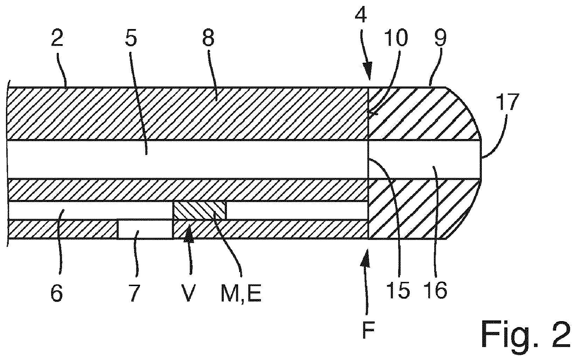

Der Katheterschlauch 2 ist zwischen einem proximalen Schlauchende 3 und einem distalen Schlauchende 4 längserstreckt und weist ein Hauptlumen 5 und wenigstens ein Nebenlumen 6 auf (Fig. 2). Das Hauptlumen 5 und das wenigstens eine Nebenlumen 6 sind jeweils zwischen dem proximalen Schlauchende 3 und dem distalen Schlauchende 4 längserstreckt. Das Nebenlumen 6 weist eine seitliche Öffnung 7 auf. Die seitliche Öffnung 7 ist in axialer Richtung des Katheterschlauchs 2 zwischen dem proximalen Schlauchende 3 und dem distalen Schlauchende 4 angeordnet. Zudem ist die seitliche Öffnung 7 in radialer Richtung des Katheterschlauchs 2 durch einen Schlauchmantel 8 des Katheterschlauchs 2 erstreckt. Die seitliche Öffnung 7 fungiert - je nach Strömungsrichtung einer mittels des Katheters 1 zu verabreichenden oder zu entnehmenden Flüssigkeit - als Austritts- und/oder Eintrittsöffnung.

Der Schlauchmantel 8 kann auch als Schlauchwandung oder Wandung bezeichnet werden.

Der Katheter 1 weist zudem einen Verschluss V auf (Fig. 2). Der Verschluss V ist in dem Nebenlumen 6 angeordnet. Dabei ist der Verschluss V in distaler Richtung hinter der seitlichen Öffnung 7 in das Nebenlumen 6 eingebracht. Mit anderen Worten ausgedrückt, ist der Verschluss V in axialer Richtung des Katheterschlauchs 2 und/oder des Nebenlumens 6 zwischen der seitlichen Öffnung 7 und dem distalen Schlauchende 4 angeordnet. Bei der gezeigten Ausführungsform ist der Verschluss V in axialer Richtung unmittelbar hinter der seitlichen Öffnung 7 an dieselbe angrenzend in das Nebenlumen 6 eingebracht.

Der Verschluss V kann gemäß der Erfindung auf unterschiedliche Weisen ausgebildet und/oder beschaffen sein:
Bei einer Ausführungsform ist der Verschluss V aus einer in viskosem Zustand in das Nebenlumen 6 eingebrachten und innerhalb des Nebenlumens 6 ausgehärteten Verschlussmasse M gebildet.

Bei einer weiteren Ausführungsform ist der Verschluss V aus einem in das Nebenlumen 6 eingebrachten und innerhalb des Nebenlumens 6 expandierten thermoplastischen Elastomermaterial E gebildet.

Fig. 2 dient zur Verdeutlichung beider Ausführungsformen. Insoweit ist Fig. 2 als schematisch stark vereinfacht aufzufassen.

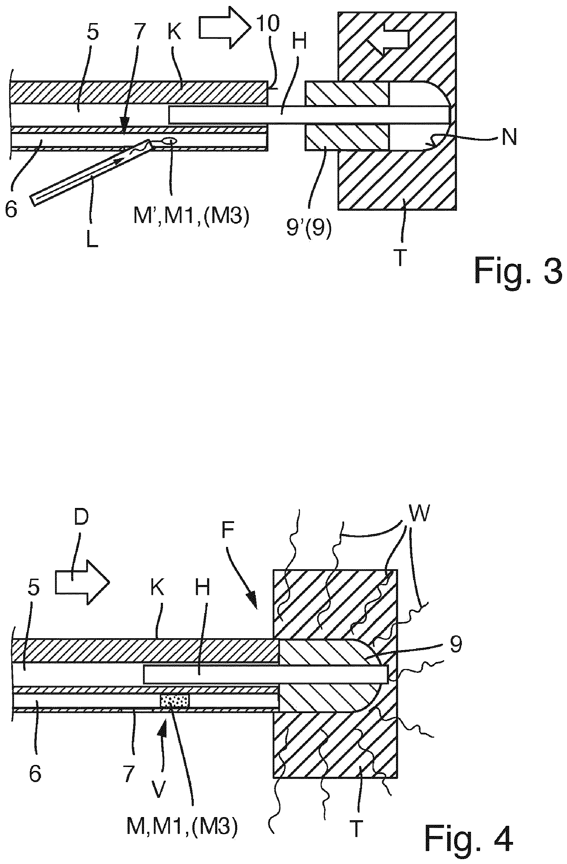

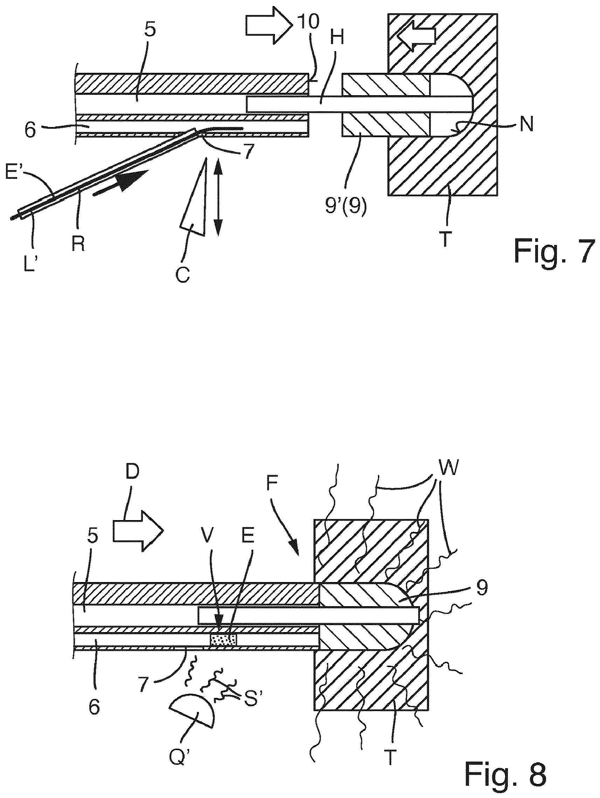

Die Ausführungsform mit ausgehärteter Verschlussmasse M wird nachfolgend noch näher anhand der Fig. 3 bis 6 erläutert. Die weitere Ausführungsform mit expandiertem thermoplastischem Elastomermaterial E wird anhand der Fig. 7 bis 9 im Detail beschrieben werden.

Nachfolgend wird zunächst auf weitere Merkmale des gezeigten Katheters 1 eingegangen, die im Hinblick auf die vorliegende Erfindung als vorteilhaft, aber nicht zwingend notwendig zu erachten sind.

Vorliegend weist der Katheter 1 eine Katheterspitze 9 auf. Die Katheterspitze 9 ist an das distale Schlauchende 4 angefügt. Vorliegend ist hierfür eine stoffschlüssige Fügeverbindung F zwischen einer stirnendseitigen Fügefläche 10 des Katheterschlauchs 2 und einem proximalen Stirnende der Katheterspitze 9 vorgesehen.

Bei einer zeichnerisch nicht dargestellten Ausführungsform weist der Katheter 1 keine Katheterspitze auf. Bei einer weiteren nicht gezeigten Ausführungsform ist die Katheterspitze nicht etwa als separat gefertigtes und hiernach an das distale Schlauchende angefügtes Bauteil, sondern stattdessen als angeformter Abschnitt des distalen Schlauchendes ausgebildet.

Im Übrigen weist der Katheter 1 abgesehen von dem erfindungsgemäß ausgestalteten Verschluss V eine dem Fachmann grundsätzlich bekannte Gestaltung und Funktionsweise auf. Dementsprechend ist das proximale Schlauchende 3 über einen Katheterhub 11 mit einer ersten Zuleitung 12 und einer zweiten Zuleitung 13 verbunden. Die beiden Zuleitungen 12, 13 weisen jeweils einen Fluidkonnektor 14 auf, der beispielsweise als Luer-Konnektor oder NRFit-Konnektor gestaltet sein kann. Die erste Zuleitung 12 ist vorliegend dem Hauptlumen 5 zugeordnet. Die zweite Zuleitung 13 ist vorliegend dem Nebenlumen 6 zugeordnet. Die beiden Zuleitungen 12, 13 sind auf grundsätzlich bekannte Weise über den Katheterhub 11 fluidleitend mit dem jeweiligen Lumen 5, 6 verbunden. Dementsprechend weist das Hauptlumen 5 eine im Bereich des proximalen Schlauchendes 3 angeordnete nicht näher gezeigte Eintrittsöffnung auf, die über den Katheterhub 11 fluidleitend mit der ersten Zuleitung 12 verbunden ist. Entsprechendes gilt sinngemäß für das Nebenlumen 6. Das Hauptlumen 5 weist im Bereich des distalen Schlauchendes 4 eine distale Austrittsöffnung 15 auf. Die distale Austrittsöffnung 15 mündet in eine nicht näher bezeichnete proximale Eintrittsöffnung der Katheterspitze 9 (Fig. 2), die über ein Lumen 16 der Katheterspitze 9 mit einer distalen Austrittsöffnung 17 der Katheterspitze 9 verbunden ist. Das Nebenlumen 6 weist keine solche distale Öffnung auf. Stattdessen ist die seitliche Öffnung 7 vorhanden. In distaler Richtung ist das Nebenlumen 6 mittels des Verschlusses V verschlossen.

Das Ausbilden des Verschlusses V aus der besagten Verschlussmasse M ist schematisch anhand der Fig. 3 bis 6 verdeutlicht. Diese Figuren beziehen sich auf einzelne Verfahrensschritte eines Verfahrens zur Herstellung des anhand der Fig. 1 und 2 gezeigten Katheters 1.

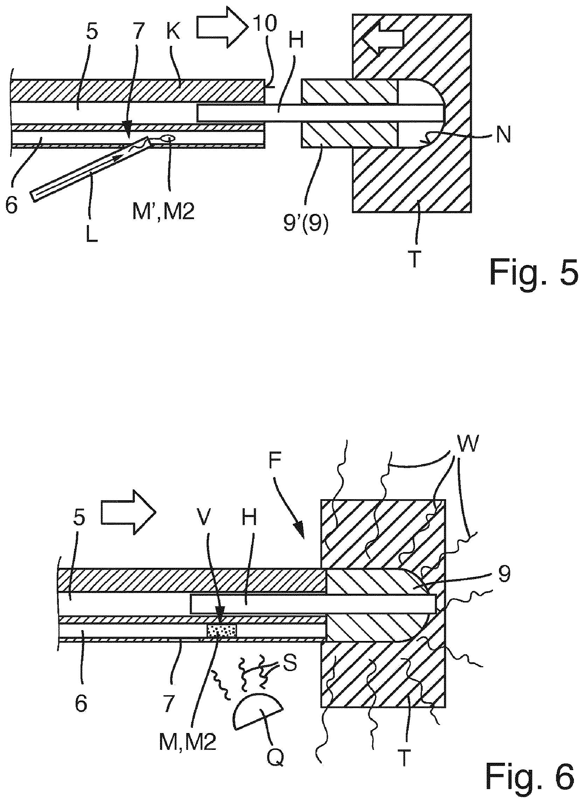

Zum Ausbilden des Verschlusses V wird die viskose Verschlussmasse M' in das Nebenlumen 6 eingebracht (Fig. 3, 5). Die viskose Verschlussmasse M' wird hierfür durch die seitliche Öffnung 7 in das Nebenlumen 6 eingebracht. Dies erfolgt vorliegend mit einem hierfür geeigneten Werkzeug L. Mittels des Werkzeugs L kann die viskose Verschlussmasse M' durch die seitliche Öffnung 7 in distaler Richtung in das Nebenlumen eingespritzt, eingeleitet, eingefüllt und/oder eingepresst werden. Bei den vorliegend gezeigten Ausführungsformen erfolgt das Einbringen der viskosen Verschlussmasse M' zusammen mit einem weiteren Schritt des Verfahrens zur Herstellung des Katheters 1. Dieser Schritt wird nachfolgend noch näher anhand der Fig. 3 und 5 erläutert und ist im Hinblick auf die vorliegende Erfindung nicht zwingend notwendig.

Nach dem Einbringen in das Nebenlumen 6 wird die (zunächst) viskose Verschlussmasse M' ausgehärtet. Nach dem Aushärten kann insoweit von einer bzw. der ausgehärteten Verschlussmasse M gesprochen werden. Das Aushärten der viskosen Verschlussmasse M' kann auf unterschiedliche Weisen erfolgen:
Die Fig. 3 und 4 beziehen sich auf eine Variante des Verfahrens, bei welcher die viskose Verschlussmasse M' unter Einfluss der Luftfeuchtigkeit aushärtet. Die Fig. 5 und 6 beziehen sich auf eine Variante des Verfahrens, bei welcher die viskose Verschlussmasse M' unter der Einwirkung einer Strahlung S (Fig. 6) ausgehärtet wird. Die beiden gezeigten Varianten unterscheiden sich dementsprechend auch im Hinblick auf die Eigenschaften, Zusammensetzung und/oder Beschaffenheit der jeweils verwendeten Verschlussmasse M:
Bei der Variante nach den Fig. 3 und 4 ist und/oder enthält die Verschlussmasse Cyanacrylat M1. Die Verwendung von Cyanacrylat M1 ermöglicht insbesondere das Aushärten unter der Einwirkung der bloßen Luftfeuchtigkeit, so dass auf eine gesonderte Strahlungseinwirkung (Fig. 6) verzichtet werden kann.

Bei einer Abwandlung der Variante nach den Fig. 3 und 4 wird anstelle des Cyanacrylats M1 Silikon M3 verwendet. Dies ist mittels des in Klammern gesetzten Bezugszeichens M3 in den Fig. 3 und 4 verdeutlicht.

Bei der Variante nach den Fig. 5 und 6 ist und/oder enthält die Verschlussmasse M ein unter der Einwirkung der Strahlung S vernetzbares und damit aushärtbares Polymer M2. Das Polymer M2 kann beispielsweise ein Harz, ein Monopolymer und/oder ein Prepolymer sein.

Bei der Strahlung S kann es - je nach Zusammensetzung, Eigenschaften und/oder Beschaffenheit des verwendeten Polymers M2 - sich um Wärmestrahlung oder UV-Licht handeln. Die Strahlung S wird mittels einer hierfür eingerichteten Strahlungsquelle Q emittiert.

Wie bereits erwähnt, sind anhand der Fig. 3 und 4 weitere Schritte zur Herstellung des Katheters 1 verdeutlicht. Diese Schritte beziehen sich auf das stoffschlüssige Anfügen der Katheterspitze 9 an das distale Schlauchende 4. Zu diesem Zweck wird die Katheterspitze 9 an einem Formwerkzeug T aufgenommen und hierzu auf eine Haltenadel H aufgefädelt. Die Haltenadel H ist einends an dem Formwerkzeug T festgelegt. Die Katheterspitze 9 weist in der anhand Fig. 3 gezeigten Konfiguration noch nicht ihre endgültige Kontur auf, so dass im Hinblick auf die Katheterspitze 9 auch von einem Halbzeug 9' gesprochen werden kann. Das Formwerkzeug T weist eine Negativform N auf, die komplementär zu der herzustellenden stirnendseitigen Kontur der Katheterspitze 9 ist. Der Katheterschlauch 2 ist mit seinem distalen Ende 4 voran ebenfalls auf die Haltenadel H aufgefädelt. Hierzu ist die Haltenadel H in proximaler Richtung in das Hauptlumen 5 eingeführt. Der Katheterschlauch 2, die Katheterspitze 9 bzw. das Halbzeug 9' und das Formwerkzeug T werden in axialer Richtung zusammengeführt. Dies ist anhand der in Fig. 3 schematisch eingezeichneten gegenläufigen Pfeile verdeutlicht.

Das Formwerkzeug T ist beheizt. Das beheizte Formwerkzeug T verursacht eine Wärmeeinwirkung W auf die Katheterspitze 9 und/oder das Halbzeug 9' und das distale Schlauchende 4. Zudem wird eine axiale Druckkraft D angelegt, mittels derer das distale Schlauchende 4 gemeinsam mit der Katheterspitze 9 in das Formwerkzeug T, genauer: dessen Negativform N, gepresst wird. Durch die Wärme- und Druckkrafteinwirkung wird zum einen die distale Endkontur der Katheterspitze 9 ausgebildet. Zusätzlich wird die Katheterspitze 9 stoffschlüssig mit der stirnendseitigen Fügefläche 10 des distalen Schlauchendes 4 zusammengefügt. Der Stoffschluss kommt durch ein Aufschmelzen, Abkühlen und Erstarren des für den Katheterschlauch 2 und/oder die Katheterspitze 9 verwendeten Werkstoffs zu Stande.

Vorliegend wird als Werkstoff ein schmelzbarer Kunststoffwerkstoff K verwendet. Die Katheterspitze 9 kann aus eben diesem schmelzbaren Kunststoffwerkstoff K oder aus einem unterschiedlichen (schmelzbaren) Kunststoffwerkstoff, mit beispielsweise geringerer Härte, gefertigt sein.

Bei der Variante des Verfahrens nach den Fig. 5 und 6 erfolgt das Anfügen der Katheterspitze 9 in entsprechender Weise, so dass zur Vermeidung von Wiederholungen auf das bereits zu den Fig. 3 und 4 Gesagte verwiesen wird.

Die Fig. 7 bis 9 beziehen sich auf diejenige Ausführungsform, bei welcher der Verschluss V aus dem expandierten thermoplastischen Elastomermaterial E gebildet ist. In einem noch nicht oder nicht vollständig expandierten Zustand ist das thermoplastische Elastomermaterial mit dem Bezugszeichen E' belegt. Bei dem expansionsfähigen thermoplastischen Elastomermaterial handelt es sich vorliegend um EP-TPE. EP-TPE ist nach Kenntnis der Erfinder jedenfalls im Bereich der Medizintechnik bislang kein geläufiger Werkstoff.

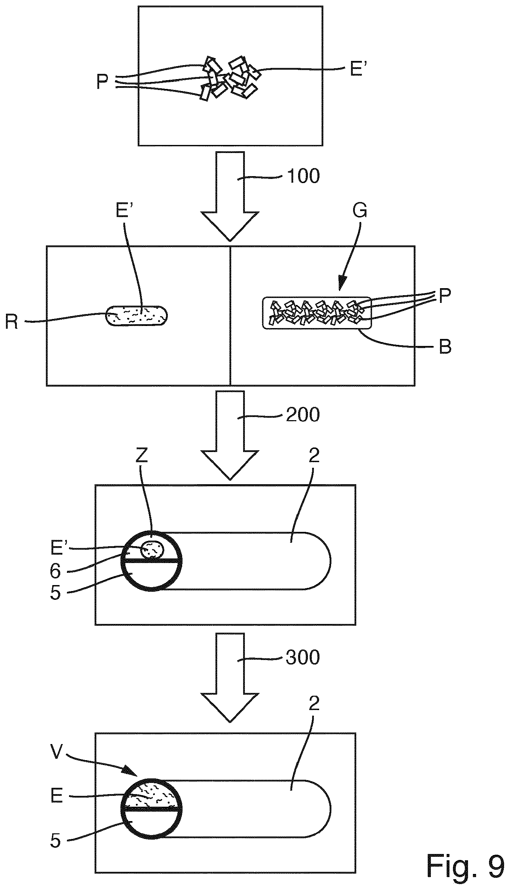

Als Grundlage für die vorliegend erörterten Verfahrensvarianten dienen anhand Fig. 9 schematisch stark vereinfacht dargestellte Partikel des expandierbaren thermoplastischen Elastomermaterials E'. Das Elastomermaterial E' und/oder die Partikel P weisen ein nicht näher bezeichnetes Treibmittel auf. Dies ist jedenfalls dem Fachmann auf dem Gebiet der Kunststofftechnik geläufig. Das besagte Treibmittel ist auf noch näher beschriebene Weise aktivierbar, wobei die Aktivierung des Treibmittels die besagte Expansion bewirkt.

Das thermoplastische Elastomermaterial E' kann in unterschiedlicher Form durch die seitliche Öffnung 7 in das Nebenlumen 6 eingebracht werden.

Bei einer Variante des Verfahrens werden die Partikel P durch die seitliche Öffnung 7 in das Nebenlumen 6 eingefüllt. Hiernach wird das Treibmittel aktiviert, die Partikel P werden expandiert und hierdurch wird der Verschluss V ausgebildet. Das Einbringen der Partikel P und das Aktivieren der Expansion sind in Fig. 9 mittels der Pfeile 200, 300 schematisch verdeutlicht.

Bei weiteren Varianten des Verfahrens ist vor dem Einbringen 200 zunächst ein Verarbeiten 100 der Partikel P vorgesehen. Dabei werden die Partikel P in unterschiedlich gestaltete und/oder beschaffene Formlinge, Halbzeuge oder dergleichen verarbeitet.

Bei einer Variante werden die Partikel P zu einer Formschnur R gepresst. Die Formschnur R ist in Fig. 9 schematisch stark vereinfacht dargestellt. Wie Fig. 7 zeigt, wird die Formschnur R mittels eines hierfür geeigneten Werkzeugs L' durch die seitliche Öffnung 7 in das Nebenlumen 6 eingebracht. Nach dem Einbringen wird die Formschnur R im Bereich der seitlichen Öffnung 7 mittels eines Schneidwerkzeugs C auf eine erforderliche und/oder gewünschte Länge eingekürzt. Sowohl das Einbringen mittels des Werkzeugs L' als auch das Abtrennen mittels des Schneidwerkzeugs C können manuell, teil- oder vollautomatisch erfolgen. Zudem ist es möglich, dass die Formschnur R bereits vor dem Einbringen auf die erforderliche und/oder gewünschte Länge eingekürzt wird. Ein nicht näher bezeichneter Querschnitt der Formschnur R ist kleiner als ein Querschnitt des Nebenlumens 6. Hierdurch kann die - noch nicht expandierte - Formschnur R ohne weiteres in axialer Richtung innerhalb des Lumens 6 vorgeschoben werden. Mit anderen Worten ausgedrückt, ist zwischen der Formschnur R und einer nicht näher bezeichneten Innenwandung des Nebenlumens 6 zunächst ein Radialspalt Z vorhanden. Der Radialspalt Z ist in Fig. 9 schematisch verdeutlicht und wird nach dem Aktivieren 300, d.h. nach dem Expandieren des thermoplastischen Elastomermaterials, vollständig verschlossen.

Bei einer weiteren Variante werden die Partikel P vor dem Einbringen 200 in eine elastische Hülle B gefüllt. Hierdurch wird ein Kissengebilde G ausgebildet. Das Kissengebilde G ist anhand Fig. 9 schematisch stark vereinfacht dargestellt. Das Kissengebilde G kann ähnlich wie die Formschnur R durch die seitliche Öffnung 7 in das Nebenlumen 6 eingeschoben werden. Hiernach erfolgt das Aktivieren und/oder Expandieren 300.

Unabhängig davon, ob das expandierbare Elastomermaterial E in Form der Partikel P, der Formschnur R oder des Kissengebildes G in das Nebenlumen 6 eingebracht wird, erfolgt das anschließende Expandieren zum Ausbilden des Verschlusses V unter Zufuhr einer Energie S' (Fig. 8). Die Energie S' wird mittels einer hierfür eingerichteten Energiequelle Q' erzeugt und dem mit dem Elastomermaterial E' versehenen Katheterschlauch 2 zugeführt. Die Energie S' wird bei unterschiedlichen Varianten des Verfahrens auf unterschiedliche Art und Weise zugeführt. Bei einer Variante wird die Energie S' als Wärme zugeführt. Die Energiequelle Q' ist in diesem Fall ein Heizelement. Denkbar ist zudem ein Erwärmen über offener Flamme. Zudem kann ein Wärmeträger, beispielsweise Wasserdampf, verwendet werden. Bei einer weiteren Variante wird die Energie S' als elektromagnetische Strahlung zugeführt. Beispielsweise kann die elektromagnetische Strahlung als Mikrowellen-, Infrarot- und/oder UV-Strahlung zugeführt werden. Um eine entsprechende Umwandlung der zugeführten elektromagnetischen Strahlung in Wärme zu ermöglichen, kann es bei Varianten des Verfahrens notwendig sein, dass dem thermoplastischen Elastomermaterial hierfür geeignete Zuschlagsstoffe beigefügt sind. Zudem kann die Energie S' mittels Induktion zugeführt werden. In diesem Fall kann dem thermoplastischen Elastomermaterial ein metallischer Anteil, beispielsweise in Form von Metallpartikeln, beigefügt sein.

Im Übrigen verdeutlichen auch die Fig. 7 und 8 das bereits erörterte Anfügen der Katheterspitze 9. Zur Vermeidung von Wiederholungen wird auf das zuvor Gesagte verwiesen.

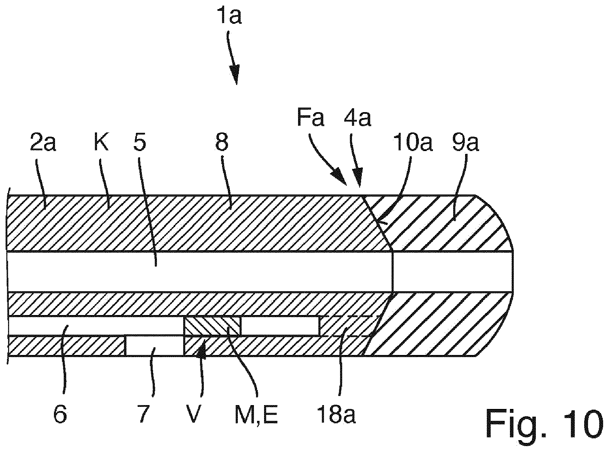

Fig. 10 zeigt eine weitere Ausführungsform eines Katheters 1a. Der Katheter 1a ist weitestgehend identisch mit dem zuvor erläuterten Katheter 1. Dementsprechend weist der Katheter 1a ebenfalls einen aus ausgehärteter Verschlussmasse M oder expandiertem thermoplastischen Elastomermaterial E gebildeten Verschluss V innerhalb des Nebenlumens 6 auf. Der Verschluss V wird auch bei dem Katheter 1a auf die zuvor erläuterte Weise und/oder unter Verwendung eines der zuvor erläuterten Verfahren ausgebildet. Zur Vermeidung von Wiederholungen werden nachfolgend lediglich wesentliche Unterschiede des Katheters 1a gegenüber dem Katheter 1 erläutert.

Wesentlicher Unterschied ist, dass der Katheter 1a im Bereich des distalen Schlauchendes 4a einen Materialpfropfen 18a aufweist. Der Materialpfropfen 18a ist dem Nebenlumen 6 zugeordnet. Der Materialpfropfen 18a ist einstückig zusammenhängend mit dem übrigen Katheterschlauch 2a, was in Fig. 10 mittels der beiden strichlierten Linien verdeutlicht ist. Mit anderen Worten ausgedrückt, ist der Materialpfropfen 18a ein integraler Bestandteil und/oder Abschnitt des Katheterschlauchs 2a. Der Materialpfropfen 18a fungiert als zusätzlicher oder weiterer Verschluss. Insoweit verschließt der Materialpfropfen 18a das Nebenlumen 6 stirnendseitig im Bereich des distalen Schlauchendes 4a. Zudem bewirkt der Materialpfropfen 18a eine mechanische Stabilisierung des distalen Schlauchendes 4a, insbesondere in radialer Richtung.

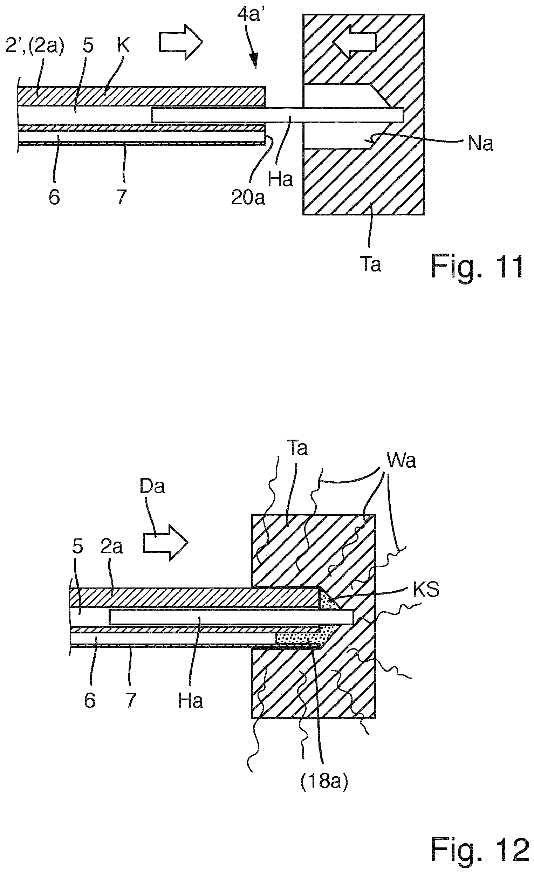

Der Materialpfropfen 18a ist durch aufgeschmolzenes und wieder erstarrtes Kunststoffmaterial K des distalen Schlauchendes 4a gebildet. Das Schmelzen und erneute Erstarren erfolgt vorliegend ausgehend von einem anhand Fig. 11 gezeigten Halbzeug 2a` zur Herstellung des Katheterschlauchs 2a, das auch als Schlauchhalbzeug bezeichnet werden kann. Dies wird nachfolgend noch im Detail beschrieben. Vereinfacht ausgedrückt wird das schmelzbare Kunststoffmaterial K im Bereich des distalen Schlauchendes des Halbzeugs 2a' unter Wärmeeinwirkung aufgeschmolzen, in proximaler Richtung in das Nebenlumen 6 des Halbzeugs 2a' eingebracht und hiernach unter Ausbildung des Materialpfropfens 18a erstarrt.

In vollständig ausgebildetem Zustand (Fig. 10) verschließt der Materialpfropfen 18a das Nebenlumen 6, genauer: dessen Querschnitt, in Radialrichtung spaltfrei. Dies gewährleistet zum einen eine vollständige Abdichtung des Nebenlumens 6 im Bereich des distalen Schlauchendes 4a. Zudem kann die bereits erwähnte verbesserte mechanische Stabilität des distalen Schlauchendes 4a in radialer Richtung erreicht werden. Dies im Unterschied zu aus dem Stand der Technik bekannten Lösungen, bei welchen das Nebenlumen im Bereich des distalen Schlauchendes mittels eines Mandrins verschlossen ist, der naturgemäß mit einem Radialspiel behaftet in das Nebenlumen eingeschoben ist.

Nachfolgend werden anhand der Fig. 11 bis 14 einzelne Schritte eines Verfahrens zur Herstellung des Katheters 1a erläutert.

Dem vorliegend verdeutlichten Verfahren liegt das in Fig. 11 gezeigte Halbzeug 2a' zugrunde. Zum Ausbilden des Materialpfropfens 18a wird das schmelzbare Kunststoffmaterial K im Bereich des distalen Schlauchendes 4a' zunächst geschmolzen. Hierfür wird vorliegend das Halbzeug 2a', genauer: dessen distales Schlauchende 4a', auf eine Haltenadel Ha aufgefädelt (Fig. 11). Die Haltenadel Ha wird hierfür in proximaler Richtung in das Hauptlumen 5 des Halbzeugs 2a` eingeführt. Die Haltenadel Ha ist einends an einem Formwerkzeug Ta befestigt. Das Formwerkzeug Ta wird zum Schmelzen des Kunststoffmaterials K beheizt. Das Halbzeug 2a` und das Formwerkzeug Ta werden in axialer Richtung zusammengeführt. Dies ist anhand der in Fig. 11 ersichtlichen entgegengesetzt orientierten Pfeile verdeutlicht. Das Halbzeug 2a` ist hierbei mittels der Haltenadel Ha in radialer Richtung geführt. Das beheizte Formwerkzeug Ta verursacht eine Wärmeeinwirkung Wa (Fig. 12). Unter der Wärmeeinwirkung Wa schmilzt das Kunststoffmaterial K im Bereich des distalen Schlauchendes 4a'. Gleichzeitig wird eine axiale Druckkraft Da auf das Halbzeug 2a' und/oder den Katheterschlauch 2a aufgebracht (Fig. 12). Unter Einwirkung der axialen Druckkraft Da und/oder des Formwerkzeugs Ta wird das geschmolzene Kunststoffmaterial in proximaler Richtung durch eine - zunächst an dem Halbzeug 2a' vorhandene distale Öffnung 20a (Fig. 11) des Nebenlumens 6 - in das Nebenlumen 6 hineingepresst. Das geschmolzene Kunststoffmaterial ist in Fig. 12 mit dem Bezugszeichen KS belegt und kann auch als Kunststoffschmelze oder Schmelzepolster bezeichnet werden.

Hiernach wird der Katheterschlauch 2a aus dem Formwerkzeug Ta entnommen. Das auf die vorbeschriebene Weise in das Nebenlumen 6 eingebrachte Kunststoffmaterial erstarrt und bildet hierdurch den besagten Materialpfropfen 18a. In der anhand Fig. 12 gezeigten Konfiguration ist die Kunststoffschmelze KS im Bereich des Nebenlumens 6 nicht oder jedenfalls noch nicht vollständig erstarrt, so dass der Materialpfropfen 18a noch nicht oder jedenfalls nicht vollständig ausgebildet ist. Das Bezugszeichen 18a ist in Fig. 12 dementsprechend in Klammern gesetzt.

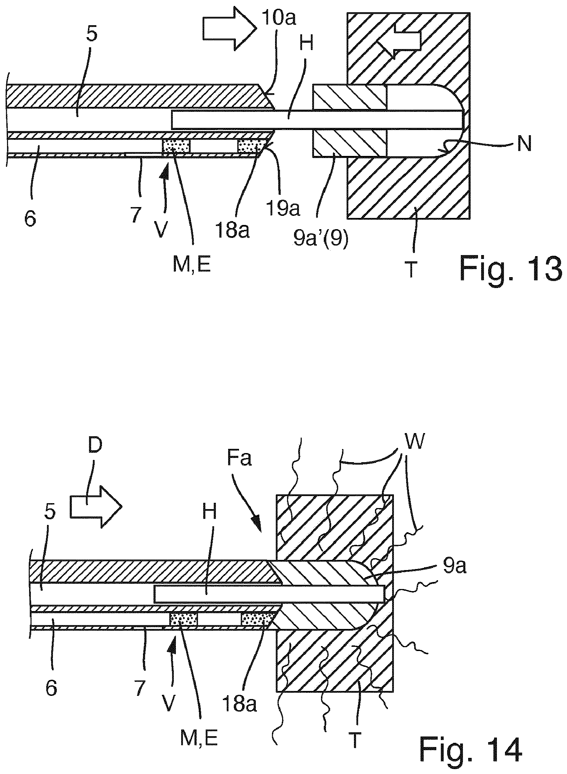

Bei der gezeigten Ausführungsform weist das Formwerkzeug Ta eine Negativform Na auf, die komplementär zu der Formgebung der stirnendseitigen Fügefläche 10a des Katheterschlauchs 2a gestaltet ist. Die stirnendseitige Fügefläche 10a ist ausgehend von der stirnendseitigen Formgebung des Halbzeugs 2a` (Fig. 11) angeschrägt. Hierdurch ergibt sich eine größere wirksame Kontaktfläche zum Ausbilden der stoffschlüssigen Fügeverbindung Fa (Fig. 14). Insoweit kann auch von einem Anschrägen des distalen Schlauchendes oder von einem angeschrägten distalen Schlauchende 4a gesprochen werden.

Nach dem Anschrägen des distalen Schlauchendes 4a wird die Katheterspitze 9a stoffschlüssig an die Fügefläche 10a angefügt (Fig. 13, 14). Die Katheterspitze 9a wird hierfür wiederum an dem bereits erörterten Formwerkzeug T aufgenommen und auf die Haltenadel H aufgefädelt.

Die Katheterspitze 9a weist in der anhand Fig. 13 gezeigten Konfiguration noch nicht ihre endgültige Kontur auf, so dass wiederum von einem Halbzeug oder Spitzenhalbzeug 9a' gesprochen werden kann. Das Ausbilden der stoffschlüssigen Fügeverbindung F zwischen dem Katheterschlauch 2a und der Katheterspitze 9 erfolgt grundsätzlich auf die bereits anhand der Fig. 3 und 4 sowie Fig. 5 und 6 erläuterte Weise. Zur Vermeidung von Wiederholungen wird insoweit auf das bereits Gesagte verwiesen.

Im Unterschied zu der stoffschlüssigen Fügeverbindung F bei dem Katheter 1 wird die stoffschlüssige Fügeverbindung Fa bei dem Katheter 1a zusätzlich zwischen dem Materialpfropfen 18a und der Katheterspitze 9a ausgebildet. Der Materialpfropfen 18a bewirkt somit gleichsam eine weitere Vergrößerung der wirksamen Fügefläche. Mit anderen Worten ausgedrückt, bildet eine distale Stirnendfläche 19a des Materialpfropfens 18a einen Abschnitt der Fügefläche 10a.

## Patentansprüche

1. Katheter (1, 1a) mit einem Katheterschlauch (2, 2a), welcher ein Hauptlumen (5) und wenigstens ein Nebenlumen (6) aufweist, wobei das Hauptlumen (5) und das wenigstens eine Nebenlumen (6) jeweils zwischen einem proximalen Schlauchende (3) und einem distalen Schlauchende (4, 4a) längserstreckt sind, und wobei das wenigstens eine Nebenlumen (6) eine seitliche Öffnung (7) aufweist, welche in axialer Richtung des Katheterschlauchs (2, 2a) zwischen dem proximalen Schlauchende (3) und dem distalen Schlauchende (4, 4a) angeordnet und in radialer Richtung des Katheterschlauchs (2, 2a) durch einen Schlauchmantel (8) des Katheterschlauchs (2, 2a) erstreckt ist, und mit einem Verschluss (V), der distal hinter der seitlichen Öffnung (7) in das Nebenlumen (6) eingebracht ist und dasselbe wenigstens teilweise verschließt, **dadurch gekennzeichnet, dass** der Verschluss (V) aus einer in viskosem Zustand in das Nebenlumen (6) eingebrachten und innerhalb des Nebenlumens (6) ausgehärteten Verschlussmasse (M) gebildet ist.

2. Katheter (1, 1a) nach Anspruch 1, **dadurch gekennzeichnet, dass** die Verschlussmasse (M) Cyanacrylat (M1) ist und/oder enthält.

3. Katheter (1, 1a) nach Anspruch 1 oder 2, **dadurch gekennzeichnet, dass** die Verschlussmasse (M) Silikon (M3) ist und/oder enthält.

4. Katheter (1, 1a) nach einem der vorhergehenden Ansprüche, **dadurch gekennzeichnet, dass** die Verschlussmasse (M) ein vernetzbares Polymer (M3), insbesondere ein Harz, ein Monopolymer und/oder ein Prepolymer, ist und/oder enthält.

5. Verfahren zur Herstellung eines Katheters (1, 1a) nach einem der vorhergehenden Ansprüche, umfassend die Schritte:
Einbringen einer viskosen Verschlussmasse (M') in das wenigstens eine Nebenlumen (6), wobei die viskose Verschlussmasse (M') durch die seitliche Öffnung (7) in das wenigstens eine Nebenlumen (6) eingebracht wird;
Aushärten der in das wenigstens eine Nebenlumen (6) eingebrachten, viskosen Verschlussmasse (M'), wobei der Verschluss (V) durch die ausgehärtete Verschlussmasse (M) gebildet wird.

6. Katheter (1, 1a) nach dem Oberbegriff des Anspruchs 1, **dadurch gekennzeichnet, dass** der Verschluss (V) aus einem in das Nebenlumen (6) eingebrachten und innerhalb des Nebenlumens (6) expandierten thermoplastischen Elastomermaterial (E) gebildet ist.

7. Verfahren zur Herstellung eines Katheters (1, 1a) nach Anspruch 6, umfassend die Schritte:
Einbringen eines expandierbaren thermoplastischen Elastomermaterials (E`) in das wenigstens eine Nebenlumen (6), wobei das expandierbare thermoplastische Elastomermaterial (E`) durch die seitliche Öffnung (7) in das wenigstens eine Nebenlumen (6) eingebracht wird;
Expandieren des in das wenigstens eine Nebenlumen (6) eingebrachten thermoplastischen Elastomermaterials (E`), wobei der Verschluss (V) durch das expandierte thermoplastische Elastomermaterial (E) gebildet wird.

8. Verfahren nach Anspruch 7, umfassend den Schritt:
Verarbeiten von Partikeln (P) des expandierbaren thermoplastischen Elastomermaterials (E'), wobei eine aus den Partikeln (P) gepresste Formschnur (R) oder ein Kissengebilde (G) mit einer elastischen und mit den Partikeln (P) befüllten Hülle (B) gebildet wird, und wobei das expandierbare thermoplastische Elastomermaterial (E`) in Form der Formschnur (R) oder des Kissengebildes (G) durch die seitliche Öffnung (7) in das wenigstens eine Nebenlumen (6) eingebracht wird.

9. Verfahren nach Anspruch 7 oder 8, wobei das Expandieren umfasst:
Aktivieren eines in dem expandierbaren Elastomermaterial (E`) enthaltenen Treibmittels, wobei das Treibmittel mittels einer Energiezufuhr (S') aktiviert wird.

10. Katheter (1a) nach einem der Ansprüche 1 bis 4 oder nach Anspruch 6, **dadurch gekennzeichnet, dass** der Katheterschlauch (2a) aus einem schmelzbaren Kunststoffmaterial (K) gefertigt ist, und dass das wenigstens eine Nebenlumen (6) im Bereich des distalen Schlauchendes (4a) mittels eines einstückig mit dem übrigen Katheterschlauch (2a) zusammenhängenden Materialpfropfens (18a) wenigstens teilweise verschlossen ist, wobei der Materialpfropfen (18a) durch aufgeschmolzenes und wieder erstarrtes Kunststoffmaterial des distalen Schlauchendes (4a) gebildet ist.

11. Katheter (1a) nach Anspruch 10, **dadurch gekennzeichnet, dass** eine Katheterspitze (9a) an das distale Schlauchende (4a) angefügt ist, wobei die Katheterspitze (9a) stoffschlüssig mit einer stirnendseitigen Fügefläche (10a) des Katheterschlauchs (2a) zusammengefügt ist, und wobei eine distale Stirnendfläche (19a) des Materialpfropfens (18a) einen Abschnitt der Fügefläche (10a) bildet.
